# EUROPÄISCHE PATENTSCHRIFT

(11) **EP 1 318 820 B1**
(45) Veröffentlichungstag und Bekanntmachung des Hinweises auf die Patenterteilung: **02.02.2005**
(21) Anmeldenummer: 01972033.3
(22) Anmeldetag: 10.09.2001
(51) Int. Cl.: A61K 33/00, A61P 9/02, A61P 25/04, A61P 25/20

(54) **ARZNEIMITTEL ENTHALTEND XENON IN SUBANÄSTHETISCHEN KONZENTRATIONEN ZUR VERWENDUNG ALS HERZ-KREISLAUFMITTEL**
MEDICAMENT CONTAINING SUB-ANESTHETIC CONCENTRATIONS OF XENON FOR USE IN THE TREATMENT OF CARDIOVASCULAR DISEASES
MEDICAMENT COMPRENANT DU XENON DANS DES CONCENTRATIONS SUB-ANESTHESIQUES POUR L'UTILISATION DANS LE TRAITEMENT DES MALADIES CARDIOVASCULAIRES

(30) Priorität: 14.09.2000 DE 10045845
(43) Veröffentlichungstag der Anmeldung: 18.06.2003
(73) Patentinhaber: Messer Griesheim GmbH, 65933 Frankfurt am Main (DE)
(72) Erfinder: HORN, Nicola, 52064 Aachen (DE); NEU, Peter, 47228 Duisburg (DE); THOMA, Klemens, 47800 Krefeld (DE); SCHUCHT, Fridtjof, 47803 Krefeld (DE); PILGER, Carsten, 47509 Rheurdt (DE); BAUMERT, Jan-Hinrich, 52072 Aachen (DE); HECKER, Klaus, 50126 Bergheim (DE); REYLE-HAHN, Matthias, 14129 Berlin (DE); ROSSAINT, Rolf, 52074 Aachen (DE); COPPIN, Arnold, B-9111 Belsele (BE)
(74) Vertreter: Münzel, Joachim R. (DE)
(86) Internationale Anmeldenummer: PCT/EP2001/010402
(87) Internationale Veröffentlichungsnummer: WO 2002/022141

(56) Entgegenhaltungen:
- EP-A- 0 523 315
- FR-A- 2 538 704
- US-A- 4 292 603
- DATABASE INSPEC [Online] INSTITUTE OF ELECTRICAL ENGINEERS, STEVENAGE, GB; KASHIWAGI S ET AL: "Measurement and imaging of cerebral blood flow with stable xenon and computed tomography (Xe-CT)" Database accession no. 2912077 XP002191541 & ELECTROMEDICA, 1986, WEST GERMANY, Bd. 54, Nr. 4, Seiten 136-144, ISSN: 0013-4724
- WEBSTER N R ET AL.: "XENON INCREASES ARTERIAL PRESSURE IN ANAESTHESIZED RATS" BRITISH JOURNAL OF ANAESTHESIA, Bd. 83, Nr. 1, Juli 1999 (1999-07), Seiten 181P-182P, XP001056315
- FRANKS N P ET AL: "HOW DOES XENON PRODUCE ANAESTHESIA?" NATURE, MACMILLAN JOURNALS LTD. LONDON, GB, Bd. 396, Nr. 6709, 26. November 1998 (1998-11-26), Seite 324 XP000960777 ISSN: 0028-0836

## Beschreibung

Die Erfindung betrifft ein Herz-Kreislaufmittel.

Xenon wird als Inhalationsanästhesiemittel verwendet. Die narkotischen Wirkungen von Xenon sind seit den 40er Jahren bekannt. Eine Mischung von etwa 80% Xenon und 20% Sauerstoff wird als nahezu ideales Narkosegas betrachtet, das gegenüber den heute überwiegend verwendeten Narkosegasen zahlreiche Vorteile hat. Eine Übersicht über die anästhetischen und pharmakologischen Eigenschaften des Xenon findet sich in F. Giunta et al., "Xenon: a review of its anaesthetic and pharmacological properties", Applied Cardiopulmonary Pathophysiology 00: 1-9, 1996.

Kreislaufmittel sind im allgemeinen Pharmaka, die Kreislaufgefäße erweitern oder verengen. Catecholamine wie Dopamin werden zur Steigerung des arteriellen Blutdrucks eingesetzt.

Herkömmliche Inhalationsanästhesiemittel mit Ausnahme von Xenon haben eine mehr oder weniger ausgeprägte Blutdruck senkende Wirkung. Bisher geht man bei Xenon davon aus, daß Xenon den Blutdruck nicht beeinflußt (vgl. Giunta et al., oben zitiert). Webster et al. ("Xenon increases arterial pressure in anaesthesized rats", British Journal of Anaesthesia, Bd. 83, Nr. 1, Juli 1999, Seiten 181P-182P) berichten von einer Erhöhung des arteriellen Druckes in zuvor mit Isofluoran anästhetisierten Ratten.

Bekannte Herz-Kreislaufmittel oder Blutdruck beeinflussende Mittel werden im Körper des Patienten metabolisiert und zeigen mehr oder weniger ausgeprägt Nebenwirkungen.

Der Erfindung liegt die Aufgabe zugrunde, ein Herz-Kreislaufmittel bereitzustellen, das weniger Nebenwirkungen aufweist.

Gegenstand der Erfindung ist die Verwendung von Xenon oder Xenon-haltigen Gasgemischen zur Herstellung eines Medikamentes mit den in Anspruch 1 beschriebenen Merkmalen.

Das Arzneimittel ist vorzugsweise gasförmig und wird vorzugsweise durch Inhalation über die Lunge verabreicht. Das Arzneimittel ist daher vorzugsweise ein Inhalationsarzneimittel.

Das Arzneimittel dient vorzugsweise als Herz-Kreislaufmittel, z. B. als Mittel zur Kreislaufstabilisierung, insbesondere als Mittel zur Blutdruckerhöhung, vorteilhaft für eine antihypotensive Therapie.

Zum Einsatz kommt das Arzneimittel in der Regel als ein die Atmung unterhaltendes Gasgemisch, das Xenon und Sauerstoff enthält.

Das bereitgestellte Arzneimittel oder das direkt bei der Anwendung, insbesondere in unmittelbarer Nähe zum Patienten, hergestellte Arzneimittel ist beispielsweise ein Gasgemisch, das 5 bis 70 Vol.-% Xenon enthält (z. B. Rest Sauerstoff). Subanästhetische Mengen von Xenon sind solche Mengen oder Konzentrationen von Xenon, die für eine Anästhesie nicht ausreichen. Das sind im allgemeinen Mengen bis zu 70 Vol.-% Xenon, vorzugsweise im Bereich von 5 bis 65 Vol.-%, besonders bevorzugt im Bereich von 25 bis 65 Vol.-%, insbesondere im Bereich von 35 bis 60 Vol.-%. Reines Xenon wird dementsprechend in den genannten Konzentrationen im Atemgas des Patienten dosiert. Das heißt das dem Patienten zugeführte Atemgas enthält beispielsweise 35 bis 60 Vol.-% oder 35 bis 55 Vol.-% Xenon. In besonderen Fällen kann eine Dosierung von Xenon in dem Atemgas mit einer niedrigen Konzentration, beispielsweise 5 bis 25 Vol.-% Xenon in dem Atemgas, vorteilhaft sein.

Die Gasgemische können neben Xenon ein oder mehrere Gase oder bei Körpertemperatur und Normaldruck gasförmige Stoffe enthalten. Verwendbare Gasgemische sind beispielsweise Xenon-Sauerstoff-Gasgemische oder Gasgemische von Xenon und einem oder mehrerer Inertgase wie Stickstoff oder einem Edelgas (z. B. Helium, Neon, Argon, Krypton) oder Xenon-Sauerstoff-Inertgas-Gasgemische. Die Beimischung eines Gases zum Xenon kann sehr vorteilhaft sein, wenn wenig Xenon in den Körper gebracht werden soll. Beispiele von Gasen oder Gasgemischen, die eingesetzt werden, folgen: 1.) 5 Vol.-% Xenon / 95 Vol.-% Sauerstoff; 2.) 70 Vol.-% Xenon / 30 Vol.-% Sauerstoff; 3.) 65 Vol.-% Xenon / 30 Vol.-% Sauerstoff / 5 Vol.-% Stickstoff; 4.) 65 Vol.-% Xenon / 35 Vol.-% Sauerstoff; 5.) 60 Vol.-% Xenon / 30 Vol.-% Sauerstoff / 10 Vol.-% Stickstoff; 6.) 60 Vol.-% Xenon / 35 Vol.-% Sauerstoff / 5 Vol.-% Stickstoff; 7.) 60 Vol.-% Xenon / 40 Vol.-% Sauerstoff; 8.) 55 Vol.-% Xenon / 25 Vol.-% Sauerstoff / 20 Vol.-% Stickstoff; 9.) 55 Vol.-% Xenon / 30 Vol.-% Sauerstoff / 15 Vol.-% Stickstoff; 10.) 55 Vol.-% Xenon / 35 Vol.-% Sauerstoff / 10 Vol.-% Stickstoff; 11.) 55 Vol.-% Xenon / 40 Vol.-% Sauerstoff / 5 Vol.-% Stickstoff; 12.) 55 Vol.-% Xenon / 45 Vol.-% Sauerstoff; 13.) 50 Vol.-% Xenon / 50 Vol.-% Sauerstoff; 14.) 50 Vol.-% Xenon / 45 Vol.-% Sauerstoff / 5 Vol.-% Stickstoff; 15.) 50 Vol.-% Xenon / 40 Vol.-% Sauerstoff / 10 Vol.-% Stickstoff; 16.) 50 Vol.-% Xenon / 30 Vol.-% Sauerstoff / 20 Vol.-% Stickstoff; 17.) 50 Vol.-% Xenon / 25 Vol.-% Sauerstoff / 25 Vol.-% Stickstoff; 18.) 45 Vol.-% Xenon / 55 Vol.-% Sauerstoff; 19.) 45 Vol.-% Xenon / 50 Vol.-% Sauerstoff / 5 Vol.-% Stickstoff; 20.) 45 Vol.-% Xenon / 45 Vol.-%. Sauerstoff / 10 Vol.-% Stickstoff; 21.) 45 Vol.-% Xenon / 40 Vol.-% Sauerstoff / 15 Vol.-% Stickstoff; 22.) 45 Vol.-% Xenon / 35 Vol.-% Sauerstoff / 20 Vol.-% Stickstoff; 23.) 45 Vol.-% Xenon / 30 Vol.-% Sauerstoff / 25 Vol.-% Stickstoff; 24.) 45 Vol.-% Xenon / 30 Vol.-% Sauerstoff / 25 Vol.-% Stickstoff; 25.) 40 Vol.-% Xenon / 30 Vol.-% Sauerstoff / 30 Vol.-% Stickstoff; 26.) 40 Vol.-% Xenon / 50 Vol.-% Sauerstoff / 10 Vol.-% Stickstoff; 27.) 35 Vol.-% Xenon / 25 Vol.-% Sauerstoff / 40 Vol.-% Stickstoff; 28.) 35 Vol.-% Xenon / 65 Vol.-% Sauerstoff; 29.) 30 Vol.-% Xenon / 70 Vol.-% Sauerstoff; 30.) 30 Vol.-% Xenon / 50 Vol.-% Sauerstoff / 20 Vol.-% Stickstoff; 31.) 30 Vol.-% Xenon / 30 Vol.-% Sauerstoff / 40 Vol.-% Stickstoff; 32.) 20 Vol.-% Xenon / 80 Vol.-% Sauerstoff; 33.) 20 Vol.-% Xenon / 30 Vol.-% Sauerstoff / 50 Vol.-% Stickstoff; 34.) 15 Vol.-% Xenon / 30 Vol.-% Sauerstoff / 55 Vol.-% Stickstoff; 35.) 15 Vol.-% Xenon / 50 Vol.-% Sauerstoff / 35 Vol.-% Stickstoff; 36.) 10 Vol.-% Xenon / 90 Vol.-% Sauerstoff; 37.) 10 Vol.-% Xenon / 50 Vol.-% Sauerstoff / 40 Vol.-% Stickstoff; 38.) 10 Vol.-% Xenon / 30 Vol.-% Sauerstoff / 60 Vol.-% Stickstoff; 39.) 10 Vol.-% Xenon / 25 Vol.-% Sauerstoff / 65 Vol.-% Stickstoff; 40.) 5 Vol.-% Xenon / 25 Vol.-% Sauerstoff / 70 Vol.-% Stickstoff; 41.) 5 Vol.-% Xenon / 30 Vol.-% Sauerstoff / 65 Vol. -% Stickstoff; 42.) 5 Vol.-% Xenon / 50 Vol.-% Sauerstoff / 45 Vol.-% Stickstoff; 43.) 5 Vol.-% Xenon / 30 Vol.-% Sauerstoff / 65 Vol.-% Stickstoff.

Xenon oder ein Xenon-haltiges Gasgemisch dienen vorzugsweise zur Herstellung eines Medikamentes zur Behandlung von Herz-Kreislauf-Instabilitäten, zur Herstellung eines gasförmigen Medikamentes zur Behandlung von Krankheiten des Herzens und/oder des Kreislaufes, zur Herstellung eines gasförmigen Medikamentes zur Behandlung von Herz-Kreislauf-Instabilitäten von Risikopatienten oder zur Herstellung eines gasförmigen Medikamentes zur Behandlung von Herz-Kreislauf-Instabilitäten bei Schilddrüsenoperationen, Lebertransplantationen, in der Gynäkologie, in der Orthopädie, in der plastischen Chirurgie oder in der Onkologie oder zur Herstellung eines Medikamentes zur Behandlung von Schock oder schockartigen Zuständen, wobei das Medikament ein Gas umfaßt, das 5 bis 70 Vol.-% gasförmiges Xenon enthält.

Weiter wird das Arzneimittel zur Behandlung von hypotonen Kreislaufzuständen, verursacht durch Herzinsuffizienz, Hypotonie, Hypovolemie oder Anästhetikumbedingter Hypotonie (z. B. einer Blutdruckabsenkung bei Narkose mit Lachgas oder einem volatilen Anästhetikum), eingesetzt.

Das Arzneimittel wird präoperativ, intraoperativ oder postoperativ eingesetzt.

Besonders vorteilhaft wird das Arzneimittel in der Intensivmedizin eingesetzt, wo z.B. über einen längeren Zeitraum ein Kreislaufmittel, Analgetikum und/oder Sedativum verabreicht werden muß, beispielsweise bei der Langzeitbeatmung und bei Polytrauma. Hier hat das Arzneimittel den besonderen Vorteil, nach derzeitigem Kenntnisstand keine Nebenwirkungen zu haben. Es bilden sich im Körper bei Verwendung von Xenon oder Xenon-haltigen Gasen als Arzneimittel keine Metabolite im Körper und es findet im Körper keine Anreicherung des Arzneimittels statt. Übliche Arzneimittel wie übliche Kreislaufmittel, Analgetika oder Sedativa können wegen auftretenden Nebenwirkungen maximal nur wenige Tage verabreicht werden und müssen dann abgesetzt werden. Die kombinierte analgetische, sedierende und kreislaufstabilisierende Wirkung des Arzneimittels ist in dieser Form bei anderen Arzneimitteln nicht bekannt.

Xenon als Inhalationsarzneimittel insbesondere zur Stabilisierung des Patienten (Mittel für Kreislauf, insbesondere für Kreislauf und Analgesie oder für Kreislauf, Analgesie und Sedierung) bei der Beatmung, insbesondere der Langzeitbeatmung, wird in der Regel als Gemisch von Xenon, Sauerstoff und gegebenenfalls einem oder mehreren weiteren Gasen (z.B. Stickstoff) eingesetzt. Xenon wird dabei in der Regel in subanästhetischen Konzentrationen im atembaren Gas (Atemgas) verabreicht. Insbesondere bei der Langzeitbeatmung ist die Verabreichung von atembaren Gasen mit einem Gehalt von 5 bis 45 Vol.-% Xenon, vorzugsweise 5 bis 40 Vol.-% Xenon, vorteilhaft. Bei der Langzeitbeatmung hat das atembare Gas beispielsweise einen Gehalt von 20 bis 30 Vol. -% Sauerstoff, wobei Sauerstoffgehalt bei Bedarf zeitweise z. B. 30 bis 95 Vol.-% Sauerstoff erhöht werden kann. Das restliche Gas in dem atembaren Gas besteht in der Regel aus Stickstoff oder einem anderen Inertgas. Xenonhaltige Gasgemische für die Beatmung bestehen beispielsweise aus 5 Vol.-% Xenon/30 Vol.-% Sauerstoff/Rest Stickstoff, 10 Vol.-% Xenon/30 Vol.-% Sauerstoff/Rest Stickstoff, 15 Vol.-% Xenon/30 Vol.-% Sauerstoff/Rest Stickstoff, 20 Vol.-% Xenon/30 Vol.-% Sauerstoff/Rest Stickstoff, 25 Vol.-% Xenon/30 Vol.-% Sauerstoff/Rest Stickstoff, 30 Vol.-% Xenon/30 Vol.-% Sauerstoff/Rest Stickstoff, 35 Vol.-% Xenon/30 Vol.-% Sauerstoff/Rest Stickstoff, 40 Vol.-% Xenon/30 Vol.-% Sauerstoff/Rest Stickstoff, 45 Vol.-% Xenon/30 Vol.-% Sauerstoff/Rest Stickstoff, 5 Vol.-% Xenon/21 Vol.-% Sauerstoff/Rest Stickstoff, 10 Vol.-% Xenon/21 Vol.-% Sauerstoff/Rest Stickstoff, 15 Vol.-% Xenon/21 Vol.-% Sauerstoff/Rest Stickstoff, 20 Vol.-% Xenon/21 Vol.-% Sauerstoff/Rest Stickstoff, 25 Vol.-% Xenon/21 Vol.-% Sauerstoff/Rest Stickstoff, 30 Vol.-% Xenon/21 Vol.-% Sauerstoff/Rest Stickstoff, 35 Vol.-% Xenon/21 Vol.-% Sauerstoff/Rest Stickstoff, 40 Vol.-% Xenon/21 Vol.-% Sauerstoff/Rest Stickstoff, 45 Vol.-% Xenon/21 Vol.-% Sauerstoff/Rest Stickstoff,5 Vol.-% Xenon/25 Vol.-% Sauerstoff/Rest Stickstoff, 10 Vol.-% Xenon/25 Vol.-% Sauerstoff/Rest Stickstoff, 15 Vol. -% Xenon/25 Vol.-% Sauerstoff/Rest Stickstoff, 20 Vol.-% Xenon/25 Vol.-% Sauerstoff/Rest Stickstoff, 25 Vol.-% Xenon/25 Vol.-% Sauerstoff/Rest Stickstoff, 30 Vol.-% Xenon/25 Vol.-% Sauerstoff/Rest Stickstoff, 35 Vol.-% Xenon/25 Vol.-% Sauerstoff/Rest Stickstoff, 40 Vol.-% Xenon/25 Vol.-% Sauerstoff/Rest Stickstoff oder 45 Vol.-% Xenon/25 Vol.-% Sauerstoff/Rest Stickstoff. Der Xenon-Gehalt des atembaren Gases kann auch vorteilhaft während der Beatmung variiert werden. Dies geschieht besonders vorteilhaft mit einem gesteuerten Gasdosiergerät, wie es z.B. in der WO 98/31282 beschrieben ist und worauf hiermit Bezug genommen wird. Beispielsweise wird mittels eines Signals eines Blutdruck-Sensors am Patienten das Gasdosiergerät gesteuert. Die Xenon-Menge in dem Atemgas kann auf diese Weise in Abhängigkeit von dem Blutdruck des Patienten dosiert werden. Die atembaren Xenon-haltigen Gasgemische werden besonders vorteilhaft in der Intensivmedizin eingesetzt.

Das eingesetzte Xenon-Gas hat im allgemeinen die natürliche Isotopenzusammensetzung. Die Isotopenzusammensetzung des Xenons kann sich von der natürlichen Isotopenzusammensetzung unterscheiden. Das Xenon-Gas wird vorzugsweise in hoher Reinheit, wie für medizinische Gase üblich, eingesetzt.

Gasförmiges Xenon (reines Xenon) wird im allgemeinen als komprimiertes Gas in Druckgasbehältern wie Druckgasflaschen oder Druckdosen bereitgestellt. Auch Xenon-haltige Gasgemische können in Druckgasbehältern bereitgestellt werden. Das gasförmige Arzneimittel kann auch in einem Behälter als verflüssigtes Gas oder Gasgemisch oder in kälteverfestigter Form bereitgestellt werden.

Das Arzneimittel wird in der Regel mit einem Beatmungsgerät mit einer Gasdosiereinheit oder mit einem Anästhesiegerät verabreicht. Das Arzneimittel wird vorteilhaft direkt zur Anwendung aus den reinen Gasen hergestellt, beispielsweise durch Zusammenmischen von Xenon, Sauerstoff und gegebenenfalls einem Inertgas (z. B. mit Hilfe eines Anästhesiegerätes) in unmittelbarer Nähe zum Patienten.

Das Arzneimittel wird in der Regel als feuchtes Gas oder wasserdampfgesättigtes Gas dem Patienten verabreicht.

## Patentansprüche

1. Verwendung von Xenon oder Xenon-haltigen Gasgemischen zur Herstellung eines Medikamentes zur Behandlung von Herz-Kreislauf-Instabilitäten beim Menschen, zur Behandlung von hypotonen Kreislaufzuständen beim Menschen, zur Stabilisierung eines menschlichen Patienten, zur Behandlung von Schock oder schockartigen Zuständen oder zur Herstellung eines gasförmigen Medikamentes zur Behandlung von Krankheiten des Herzens und/oder des Kreislaufes beim Menschen, wobei das Medikament ein Gas umfaßt, das 5 bis 70 Vol.-%, 5 bis 65 Vol.-%, 25 bis 65 Vol.-%, 35 bis 60 Vol.-% oder 5 bis 25 Vol.-% gasförmiges Xenon enthält.

2. Verwendung nach Anspruch 1, **dadurch gekennzeichnet, dass** die hypotonen Kreislaufzustände durch Herzinsuffizienz, Hypotonie, Hypovolemie oder Anästhetika bedingter Hypotonie verursacht sind.

3. Verwendung nach Anspruch 1 oder 2, **dadurch gekennzeichnet, dass** das Medikament Helium, Neon, Argon oder Krypton enthält.

## Claims

1. Use of xenon or xenon containing gas mixtures for producing a medicinal product for the treatment of cardiovascular instabilities in humans, for the treatment of hypotensive circulation conditions in humans, for stabilizing a human patient, for the treatment of shock or shock-like conditions or for producing a gaseous medicinal product for the treatment of diseases of the heart and/or of the vascular system in humans, where the medicinal product comprises a gas, that contains 5 vol.-% to 70 vol.-%, 5 vol.-% to 65 vol.-%, 25 vol.-% to 65 vol.-%, 35 vol.-% to 60 vol.-%, or 5 vol.-% to 25 vol.-% of gaseous xenon.

2. Use according to claim 1, **characterized in that** the hypotensive circulation conditions are caused by cardiac insufficiency, hypotension, hypovolemia or anesthetic-induced hypotension.

3. Use according to claim 1 or 2, **characterized in that** the medicinal product contains helium, neon, argon or krypton.

## Revendications

1. Utilisation de xénon ou de mélanges gazeux contenant du xénon pour la préparation d'un médicament pour le traitement d'instabilités cardio-circulatoires chez l'homme, pour le traitement d'états circulatoires hypotoniques chez l'homme, pour la stabilisation d'un patient humain, pour le traitement de choc ou d'états de choc ou pour la préparation d'un médicament sous forme gazeuse pour le traitement de maladies du coeur et/ou de la circulation chez l'homme, le médicament comprenant un gaz qui contient de 5 à 70 % en volume, de 5 à 65 % en volume, de 25 à 65 % en volume, de 35 à 60 % en volume ou de 5 à 25 en volume de xénon sous forme gazeuse.

2. Utilisation selon la revendication 1, **caractérisée en ce que** les états circulatoires hypotoniques sont provoqués par une insuffisance cardiaque, une hypotonie, une hypovolémie ou une hypotonie causée par une anesthésie.

3. Utilisation selon la revendication 1 ou 2, **caractérisée en ce que** le médicament contient de l'hélium, du néon, de l'argon ou du krypton.
